# EUROPÄISCHE PATENTSCHRIFT

(11) **EP 3 697 248 B1**
(45) Veröffentlichungstag und Bekanntmachung des Hinweises auf die Patenterteilung: **09.09.2026**
(21) Anmeldenummer: 18800832.0
(22) Anmeldetag: 19.10.2018
(51) Int. Cl.: A61F 5/14, A43B 3/26, A43B 7/1415

(54) **EINLAGENSOHLENELEMENT MIT PELOTTE**
INSOLE ELEMENT WITH PAD
ÉLÉMENT DE SEMELLE INTÉRIEURE AYANT UNE PELOTE

(30) Priorität: 20.10.2017 DE 102017009810
(43) Veröffentlichungstag der Anmeldung: 26.08.2020
(73) Patentinhaber: Adomus GmbH, 4710 Grieskirchen (AT)
(72) Erfinder: STOCKINGER, Hannes, 4710 Grieskirchen (AT)
(74) Vertreter: Burgstaller, Peter
(86) Internationale Anmeldenummer: PCT/AT2018/060248
(87) Internationale Veröffentlichungsnummer: WO 2019/075501

(56) Entgegenhaltungen:
- DE-U1- 29 810 515
- DE-U1- 29 911 499
- US-A- 5 551 173

## Beschreibung

Die Erfindung betrifft ein Einlagesohlenelement mit einer Flächenausdehnung für eine Gruppe von Schuhgrößen umfassend eine Klimaschicht und eine Pelotte. Einlagesohlen nach dem Stand der Technik umfassen im Sinne der Anordnung von oben nach unten eine Decke, eine Klimaschicht und einen Trägerkern. Zwischen der Decke und der Klimaschicht wird eine Pelotte angeordnet, um das Sohlenbett in dem Pelottenbereich zu formen. Durch das Anordnen einer Pelotte in einer Sohle wird eine Wölbung des Fußes im Bereich der Pelotte erreicht.

US5551173A offenbart ein Einlagesohlenelement mit einer Flächenausdehnung für eine Gruppe von Schuhgrößen umfassend eine Pelotte aber offenbart nicht die Merkmale des kennzeichnenden Teils des Anspruches 1.

DE29810515U1 offenbart ein Einlagesohlenelement mit einer Flächenausdehnung für eine Gruppe von Schuhgrößen umfassend eine Pelotte aber offenbart nicht die Merkmale des kennzeichnenden Teils des Anspruches 1.

DE29911499U1 offenbart ein Einlagesohlenelement mit einer Flächenausdehnung für eine Gruppe von Schuhgrößen umfassend eine Pelotte aber offenbart nicht die Merkmale des kennzeichnenden Teils des Anspruches 1.

Bei Herstellungsverfahren von Einlagesohlen wird die Pelotte auf die Klimaschicht geklebt. Der Schuhmacher bringt hierzu selbst Markierungen an, an welche er die Pelotte aufkleben möchte, oder positioniert die Pelotte überhaupt nach Gefühl. Dieser Fertigungsprozess, welcher nach dem Stand der Technik händisch durchgeführt wird, ist stark dem mehr oder weniger tagesabhängigen Geschick des Schuhmachers unterworfen. In Abhängigkeit davon wie ungenau oder unsauber die Pelotte auf die Klimaschicht aufgebracht wurde, ist die Einlagesohle durch Schleifen nachzubearbeiten.

Da das Herstellungsverfahren für eine Einlagesohle derart stark dem Geschick des Schuhmachers unterworfen ist, ist im Grunde keine Reproduzierbarkeit der Einlagesohle gegeben.

Die im Folgenden diskutierte Erfindung stellt sich im Allgemeinen die Aufgabe, den Fertigungsprozess eines Einlagesohlenelementes umfassend eine Klimaschicht und eine Pelotte so zu gestalten, dass das Einlagesohlenelement eine höhere Genauigkeit aufweist und mit einer gleichbleibenden Qualität reproduzierbar ist.

Diese Aufgabe wird durch ein Einlagesohlenelement gemäß den Ansprüchen gelöst.

Das erfindungsgemäße Einlagensohlenelement zeichnet sich dadurch aus, dass die Klimaschicht und die Pelotte einstückig ausgebildet sind,
wobei das Einlagesohlenelement eine größere Flächenausdehnung als eine Sollsohle aufweist, sodass die Pelotte durch Zuschneiden des Einlagesohlenelementes auf eine Sollsohlengröße an einer Pelottenposition für die Gruppe von Schuhgrößen anordenbar ist.

Die erfindungsgemäße Lösung basiert auf einer Abänderung des Aufbaues einer Einlagesohle durch das Bereitstellen des erfindungsgemäßen Einlagesohlenelementes, welches mit einer Decke und einem Trägerkern kombiniert werden kann. Eine Einlagesohle umfassend ein erfindungsgemäßes Einlagesohlenelement unterscheidet sich von einer Einlagesohle nach dem Stand der Technik dadurch, dass Pelotte und Klimaschicht einstückig vorliegen. Die Klimaschicht dient nun auch als Pelottenträgerschicht.

Durch das Ausbilden des erfindungsgemäßen Einlagesohlenelements umfassend die Klimaschicht und die Pelotte als ein Werkstück sind die wesentlichen konstruktiven Elemente einer Einlagesohle in einem Werkstück vereint. Das Einlagesohlenelement ist als ein Werkstück durch maschinelle Prozesse herstellbar.

Die Flächenausdehnung einer Sollsohle ist durch einschlägige Normen geregelt, welche Normen der Fachmann kennt. Der Fachmann ist in der Lage, die Flächenausdehnung so zu gestalten, dass ein Zuschneiden des Einlagesohlenelementes auf die größte Schuhgröße der Gruppe von Schuhgrößen unter Positionierung der Pelotte an einer Pelottenposition möglich ist.

Das erfindungsgemäße Einlagesohlenelement kann eine Klimaschicht aus PUR-Schaum und eine Pelotte aus PUR-Schaum umfassen, wobei die Klimaschicht und die Pelotte durch Verpressen mittels eines Formstückes unter Wärme als ein Werkstück herstellbar sind.

Der Fachmann erhält die notwendigen Angaben über den Druck und die Temperatur vom Hersteller eine PUR-Schaumes.

Das Verpressen von Klimaschicht und Pelotte kann unter der Verwendung von Formstücken erfolgen, welche Formstücke auf das Einlagesohlenelement gepresst werden. Hierdurch können die Form der Einlagesohle und die Härte des Schaumes in Teilbereichen der Einlagesohle vorgegeben werden.

Ergänzend oder alternativ zum Pressen des PUR-Schaumes unter Wärme können die Klimaschicht aus PUR-Schaum und die Pelotte aus PUR-Schaum durch Schäumen in einem Formstück als ein Werkstück hergestellt werden.

Die Klimaschicht und die Pelotte können durch ein Haftmittel und/oder durch ein Verbundmittel zu einem Werkstück verbunden werden.

Das Einlagesohlenelement umfassend die Klimaschicht und die auf der Klimaschicht angeordnete Pelotte kann durch Formen unter Druck und/oder Wärme in eine Sollform gebracht werden.

Nach dem Stand der Technik können auf die Klimaschicht und die Pelotte eine Decke aufgebracht werden, um den Komfort für den Benutzer weiter zu erhöhen. Hierbei ist es entscheidend, dass die Decke richtig und genau auf der darunter zu liegen kommenden Klimaschicht positioniert wird.

Erfindungsgemäß wird dies dadurch erreicht, dass das Einlagesohlenelement an einer Oberfläche eine erste Zentrierlinie zum Positionieren einer Decke umfasst, welche Zentrierlinie sich von einem Fersenbereich in die Pelotte erstreckt.

Die Decke kann hierzu eine zweite Zentrierlinie oder andere Markierungen umfassen, sodass der Fachmann beim Positionieren der Decke über dem Einlagesohlenelement die Zentrierlinien lediglich deckungsgleich anordnen muss.

Das erfindungsgemäße Einlagesohlenelement kann an einer Oberfläche eine Maßangabe zum Positionieren einer Decke umfassen.

Es ist hierbei entscheidend, dass die Decke so über dem Einlagensohlenelement angeordnet wird, dass die Pelotte in der geforderten Pelottenposition der Sohle angeordnet wird. Durch die Maßangabe kann eine Position einer Kante der Decke definiert werden.

Die Maßangabe kann einen Abstand vom Rand des Einlagesohlenelementes angeben.

Das Einlagesohlenelement kann an einer Oberfläche Markierungen zum Positionieren einer Decke umfassen, welche Markierungen an die Form einer Kante der auf die Oberfläche aufzubringenden Decke angepasst sind. Hierdurch wird einmal mehr sichergestellt, dass die Decke richtig auf dem Einlagensohlenelement positioniert wird.

Das Einlagesohlenelement kann an einer Oberfläche ein Haftmittel oder ein Verbundmittel zum Aufbringen einer Decke auf das Einlagesohlenelement umfassen.

Die Erfindung betrifft auch eine Einlagesohle umfassend ein Einlagesohlenelement mit einer Decke gemäß obiger Beschreibung. Eine solche Einlagesohle kann an einen Schuhmacher ausgeliefert werden, welcher Schuhmacher nach den weiteren Wünschen des Kunden einen Trägerkern auf die Einlagesohle aufbringt.

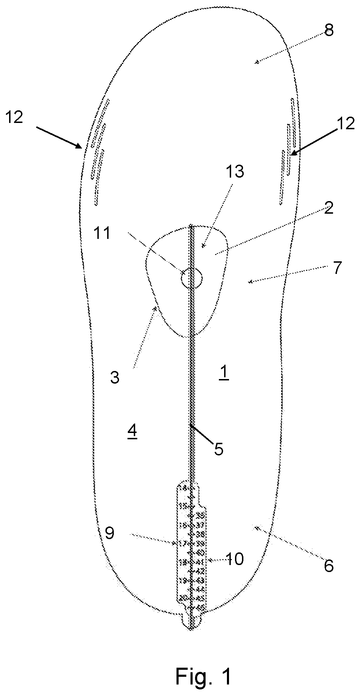

Zum besseren Verständnis der Erfindung wird diese anhand der nachfolgenden Figuren näher erläutert, wobei die in der Bezugszeichenliste angeführten Bezugszeichen in der Figur 1 die nachstehenden Elemente kennzeichnen.

Figur 1 zeigt eine Ansicht einer Ausführungsform eines erfindungsgemäßen Einlagesohlenelementes.

### FIGURENBESCHREIBUNG

Figur 1 zeigt eine Ansicht einer Ausführungsform eines erfindungsgemäßen Einlagesohlenelementes umfassend eine Klimaschicht 1 und eine Pelotte 2. Die Klimaschicht 1 erstreckt sich bei dem in Figur 1 gezeigten Ausführungsbeispiel über die gesamte Flächenausdehnung des Einlagesohlenelementes.

Die Pelotte 2 ist in der Flächenausdehnung des Einlagesohlenelementes angeordnet.

Die Klimaschicht 1 ist aus einem ersten PUR-Schaum und die Pelotte 2 ist aus einem zweiten PUR-Schaum hergestellt. Durch Verpressen der Klimaschicht und der Pelotte unter Wärme gemäß Angaben des Herstellers von PUR-Schäumen wird aus dem ersten PUR-Schaum und dem zweiten PUR-Schaum ein einstückiges Werkstück hergestellt. Das mittels geeigneten Vorrichtungen durchführbare Verpressen unter Wärme zu einem Werkstück hat den Vorteil, dass insbesondere der Kantenbereich 3 der Pelotte 2 fest mit der Klimaschicht 1 verklebt wird, was bei Einlagesohlenelementen nach dem Stand der Technik oft nicht der Fall ist.

Die Pelotte 2 stellt wie bei Einlagesohlen nach dem Stand der Technik eine Erhebung in der Ebene des Einlagesohlenelementes dar. Es ist Aufgabe der Erfindung, Maßnahmen bereitzustellen, dass die durch die Pelotte definierte Erhebung in der Ebene des Einlagesohlenelementes in der herzustellenden Sohle richtig positioniert ist.

Die Umrisse der herzustellenden Sohle werden durch eine Decke definiert, welche Decke auf das erfindungsgemäße Einlagensohlenelement aufgebracht wird. Die erfindungsgemäßen Aufgabenstellungen werden im Wesentlichen durch das genaue Positionieren der Decke gelöst.

Das Einlagesohlenelement umfasst zum genauen Positionieren einer in Figur 1 nicht dargestellten Decke an der Oberfläche 4, auf welche Oberfläche 4 die Decke aufgebracht werden soll, eine erste Zentrierlinie 5. Die erste Zentrierlinie 5 erstreckt sich im Wesentlichen parallel zu einer in Figur 1 nicht eingetragenen Längsachse des Einlagesohlenelementes. Zum Positionieren der Decke kann die Decke parallel zu der ersten Zentrierlinie 5 angeordnet werden. Die in Figur 1 nicht dargestellte Decke kann hierzu eine zweite Zentrierlinie umfassen, welche sich analog zu der ersten Zentrierlinie 5 parallel zu der Längsachse der Decke erstreckt.

Die erste Zentrierlinie 5 erstreckt sich ausschließlich im Fersenbereich und Mittelbereich des erfindungsgemäßen Einlagesohlenelementes, da in diesem hinteren Bereich die Ausrichtung der Decke entscheidend ist. Da der Spitz einer herzustellenden Sohle nicht immer in der Mitte dieser Sohle ist, wäre eine Erstreckung der ersten Zentrierlinie 5 in den Spitzenbereich 8 des Einlagesohlenelementes nicht vorteilhaft, weil dies den Schuhmacher nur verwirren würde.

Die erste Zentrierlinie 5 erstreckt sich vom Fersenbereich 6 in die Pelotte 2.

Das Einlagesohlenelement umfasst weiters an der Oberfläche 4 eine erste Maßangabe 9 und eine zweite Maßangabe 10 zum Positionieren einer Decke. Die erste Maßangabe 9 gibt einen Abstand von einem Hochpunkt wie beispielsweise den Hochpunkt der höchsten Erhebung 11 der Pelotte 2 an. Der Fachmann ist sohin in der Lage die hintere Kante der aufzubringenden Decke bei einer Maßangabe zu positionieren, wodurch die exakte Beabstandung des Hochpunktes 11 von der hinteren Kante der Sohle und insgesamt die exakte Positionierung des Hochpunktes 11 in der Sohle erreicht wird.

Das erfindungsgemäße Einlagensohlenelement umfasst weiters eine zweite Maßangabe 10, welche genormte Schuhgrößenangaben beinhaltet. Der Fachmann kann zum exakten Positionieren der Decke die hintere Kante der Decke bei einer zweiten Maßangabe 10 positionieren, um so eine Positionierung des Hochpunktes 11 in der herzustellenden Sohle in Abhängigkeit der Schuhgröße zu erreichen.

Das erfindungsgemäße Einlagesohlenelement umfasst weiters an der Oberfläche 4 Markierungen 12 zum Positionieren einer Decke, welche Markierungen 12 an die Form einer Kante der auf die Oberfläche aufzubringenden Decke oder an die Form der herzustellenden Sohle angepasst sind. Durch diese Markierungen 12 ist der Fachmann abermals in der Lage, die Decke exakt auf dem Einlagesohlenelement zu positionieren.

Die in Figur 1 dargestellte Ausführungsform des erfindungsgemäßen Einlagesohlenelementes ist gemäß zweiter Maßangabe für die genormten Schuhgrößen 36 bis 46 geeignet. Nachdem die Ausrichtung der Decke zu dem Einlagesohlenelement durch die Markierungen 12 und die erste Zentrierlinie 5 vorgegeben ist, ist der Fachmann zweifelslos in der Lage, die Flächenausdehnung des Einlagesohlenelementes so zu dimensionieren, dass das Einlagesohlenelement eine größere Flächenausdehnung als eine Sollsohle mit der Normgröße 46 aufweist. Hierdurch wird sichergestellt, dass die Pelotte durch Zuschneiden des Einlagesohlenelementes auf eine Sollsohlengröße in einer Pelottenposition 13 für die Gruppe von Normschuhgrößen 36 bis 46 anordenbar ist.

Das Einlagesohlenelement umfasst an der Oberfläche 4 eine Klebeschicht als Haftmittel zum Aufbringen einer Decke auf die Oberfläche 4 des Einlagesohlenelementes.

Das in Figur 1 gezeigte Ausführungsbeispiel zeigt eine mögliche Ausführungsform, wobei an dieser Stelle bemerkt sei, dass die Erfindung nicht auf diese dargestellte Ausführungsvariante eingeschränkt ist, sondern vielmehr auch diverse Abänderungen dieser Ausführungsform möglich sind und diese Variationsmöglichkeit aufgrund der Lehre zum technischen Handeln durch die gegenständliche Erfindung im Können des auf diesem technischen Gebiet tätigen Fachmannes liegt.

Der Schutzbereich ist durch die Ansprüche bestimmt. Die Beschreibung und die Zeichnungen sind jedoch zur Auslegung der Ansprüche heranzuziehen. Einzelmerkmale oder Merkmalskombinationen aus den gezeigten und beschriebenen unterschiedlichen Ausführungsbeispielen können für sich eigenständige erfinderische Lösungen darstellen. Die den eigenständigen erfinderischen Lösungen zugrundeliegende Aufgabe kann der Beschreibung entnommen werden.

Der Ordnung halber sei abschließend darauf hingewiesen, dass zum besseren Verständnis des Aufbaus Elemente teilweise unmaßstäblich und/oder vergrößert und/oder verkleinert dargestellt wurden.

### Bezugszeichenliste

- 1: Klimaschicht
- 2: Pelotte
- 3: Kantenbereich Pelotte
- 4: Oberfläche
- 5: Erste Zentrierlinie
- 6: Fersenbereich
- 7: Mittelbereich
- 8: Spitzenbereich
- 9: Erste Maßangabe
- 10: Zweite Maßangabe
- 11: Hochpunkt
- 12: Markierungen
- 13: Pelottenposition

## Patentansprüche

1. Einlagesohlenelement mit einer Flächenausdehnung für eine Gruppe von Schuhgrößen umfassend
- eine Klimaschicht (1),
- eine Pelotte (2),
wobei die Klimaschicht (1) und die Pelotte (2) einstückig ausgebildet sind,
wobei das Einlagesohlenelement eine größere Flächenausdehnung als eine Sollsohle aufweist, sodass die Pelotte (2) durch Zuschneiden des Einlagesohlenelementes auf eine Sollsohle an einer Pelottenposition (13) für die Gruppe von Schuhgrößen anordenbar ist,
**dadurch gekennzeichnet, dass**
das Einlagesohlenelement an einer Oberfläche (4) eine erste Zentrierlinie (5) zum deckungsgleichen Positionieren einer zweiten Zentrierlinie einer Decke umfasst, welche erste Zentrierlinie (5) sich von einem Fersenbereich (6) in die Pelotte (2) erstreckt.

2. Einlagesohlenelement nach Anspruch 1, **dadurch gekennzeichnet, dass** das Einlagesohlenelement an der Oberfläche (4) eine erste Maßangabe (9) zum Positionieren der Decke umfasst, welche erste Maßangabe (9) einen Abstand von einem Punkt (11) der Pelotte angibt.

3. Einlagesohlenelement nach einem der Ansprüche 1 bis 2 mit einer Decke, **dadurch gekennzeichnet, dass**
das Einlagesohlenelement an einer Oberfläche (4) sich über nur einen Teilbereich der Oberfläche (4) erstreckende Markierungen (12) zum Positionieren der Decke umfasst, welche Markierungen (12) an die Form einer Kante der auf die Oberfläche aufzubringenden Decke angepasst sind.

4. Einlagesohlenelement nach einem der Ansprüche 1 bis 3, **dadurch gekennzeichnet, dass**
die Klimaschicht (1) aus PUR-Schaum und die Pelotte (2) aus PUR-Schaum durch Verpressen mittels einer Form unter Wärme als ein Werkstück hergestellt sind.

5. Einlagesohlenelement nach einem der Ansprüche 1 bis 4, **dadurch gekennzeichnet, dass**
die Klimaschicht (1) aus PUR-Schaum und die Pelotte (2) aus PUR-Schaum durch Schäumen in einer Form als ein Werkstück hegestellt sind.

6. Einlagesohlenelement nach einem der Ansprüche 1 bis 5, **dadurch gekennzeichnet, dass**
die Klimaschicht (1) und die Pelotte (2) durch ein Haftmittel und/oder durch ein Verbundmittel zu einem Werkstück verbunden sind.

7. Einlagesohlenelement nach einem der Ansprüche 1 bis 6, **dadurch gekennzeichnet, dass**
das Einlagesohlenelement durch Formen unter Druck und/oder Wärme in eine Sollform bringbar ist.

8. Einlagesohlenelement nach einem der Ansprüche 1 bis 7, **dadurch gekennzeichnet, dass**
das Einlagesohlenelement an einer Oberfläche (4) eine weitere erste Maßangabe (9) zum Positionieren einer Decke umfasst,
welche weitere erste Maßangabe (9) einen Abstand vom Rand des Einlagesohlenelementes angibt.

9. Einlagesohlenelement nach einem der Ansprüche 1 bis 8, **dadurch gekennzeichnet, dass**
das Einlagesohlenelement an einer Oberfläche (4) ein Haftmittel oder ein Verbundmittel zum Aufbringen einer Decke auf die Oberfläche (4) des Einlagesohlenelementes umfasst.

10. Einlagesohlenelement nach einem der Ansprüche 1 bis 9, **dadurch gekennzeichnet, dass**
das Einlagesohlenelement an einer Oberfläche (4) ein Haftmittel oder ein Verbundmittel zum Aufbringen eines Trägerkerns auf die Oberfläche (4) des Einlagesohlenelementes umfasst.

11. Einlagesohle umfassend ein Einlagesohlenelement und eine auf das Einlagesohlenelemente aufgebrachte Decke nach einem der Ansprüche 1 bis 10.

12. Einlagesohle nach Anspruch 11, **dadurch gekennzeichnet, dass** die Decke eine zweite Zentrierlinie umfasst.

## Claims

1. An inlay sole element having an areal extent for a group of shoe sizes, comprising:
- a climatizing layer (1),
- a truss pad (2),
the climatizing layer (1) and the truss pad (2) being formed integrally,
wherein the inlay sole element has an areal extent larger than a desired sole, so that the truss pad (2) can be arranged in a truss pad position (13) for the group of shoe sizes by cutting the inlay sole element to the size of a desired sole, **characterised in that**
the inlay sole element comprises, on a surface (4), a first centring line (5) for congruent positioning a second centring line of a cover, which first centring line (5) extends from a heel region (6) into the truss pad (2).

2. The inlay sole element of claim 1, **characterised in that**
the inlay sole element comprises, on the surface (4), a first measurement (9) for positioning the cover, which first measurement (9) indicates a distance from a point (11) on the truss pad.

3. The inlay sole element of any one of claims 1 to 2 having a cover, **characterised in that**
the inlay sole element comprises, on a surface (4), marks (12) extending over a mere partial region of the surface (4) for positioning the cover, which marks (12) are adapted to the shape of an edge of the cover to be applied to the surface.

4. The inlay sole element of any one of claims 1 to 3, **characterised in that**
the climatizing layer (1) made of polyurethane foam and the truss pad (2) made of polyurethane foam are fabricated as one workpiece by injecting under heat using a mould.

5. The inlay sole element of any one of claims 1 to 4, **characterised in that**
the climatizing layer (1) made of polyurethane foam and the truss pad (2) made of polyurethane foam are fabricated as one workpiece by foaming.

6. The inlay sole element of any one of claims 1 to 5, **characterised in that**
the climatizing layer (1) and the truss pad (2) are connected to make one workpiece by adhesive and/or composite means.

7. The inlay sole element of any one of claims 1 to 6, **characterised in that**
the inlay sole element can be brought into a desired shape by moulding under pressure and/or heat.

8. The inlay sole element of any one of claims 1 to 7, **characterised in that**
the inlay sole element comprises, on a surface (4), another first measurement (9) for positioning a cover,
which other first measurement (9) indicates a distance from the edge of the inlay sole element.

9. The inlay sole element of any one of claims 1 to 8, **characterised in that**
the inlay sole element comprises, on a surface (4) adhesive or composite means for applying a cover onto the surface (4) of the inlay sole element.

10. The inlay sole element of any one of claims 1 to 9, **characterised in that**
the inlay sole element comprises, on a surface (4) adhesive or composite means for applying a core onto the surface (4) of the inlay sole element.

11. An inlay sole comprising an inlay sole element and a cover applied onto said inlay sole element of any one of claims 1 to 10.

12. The inlay sole of claim 11, **characterised in that**
the cover comprises a second centring line.

## Revendications

1. Élément de semelle intérieure présentant une surface couvrant une plage de pointures, comprenant
- une couche de climatisation (1),
- une pelote (2),
la couche de climatisation (1) et la pelote (2) étant formées d'une seule pièce, l'élément de semelle intérieure présentant une surface plus grande que celle d'une semelle de référence, de sorte que la pelote (2) puisse être positionnée à un emplacement de pelote (13) correspondant une plage de pointure, en découpant l'élément de semelle intérieure à la taille d'une semelle de référence,
**caractérisé en ce que**
l'élément de semelle intérieure comporte, sur une surface (4), une première ligne de centrage (5) permettant de positionner en coïncidence une deuxième ligne de centrage d'une couverture,
ladite première ligne de centrage (5) s'étendant depuis une zone du talon (6) jusqu'à la pelote (2).

2. Élément de semelle intérieure selon la revendication 1, **caractérisé en ce que** l'élément de semelle intérieure comporte, sur sa surface (4), une première indication dimensionnelle (9) pour positionner la couverture, ladite première indication dimensionnelle (9) indiquant une distance par rapport à un point (11) de la pelote.

3. Élément de semelle intérieure selon l'une des revendications 1 à 2 avec une couverture, **caractérisé en ce que**
l'élément de semelle intérieure comporte, sur une surface (4), des repères (12) s'étendant uniquement sur une partie de ladite surface (4) et destinés au positionnement de la couverture, lesdits repères (12) étant adaptés à la forme d'un bord de la couverture à poser sur ladite surface.

4. Élément de semelle selon l'une des revendications 1 à 3, **caractérisée en ce que** la couche de climatisation (1) en mousse PUR et la pelote (2) en mousse PUR sont fabriquées d'une seule pièce par moulage à chaud ent utilisant un moule.

5. Élément de semelle selon l'une des revendications 1 à 4, **caractérisée en ce que** la couche de climatisation (1) en mousse PUR et la pelote (2) en mousse PUR sont fabriquées d'une seule pièce par moussage dans un moule.

6. Élément de semelle selon l'une des revendications 1 à 5, **caractérisée en ce que** la couche de climatisation (1) et la pelote (2) sont assemblées en une seule pièce à l'aide d'un adhésif et/ou d'un agent de liaison.

7. Élément de semelle selon l'une des revendications 1 à 6, **caractérisée en ce que** l'élément de semelle intérieure peut être mis en une forme souhaitée par moulage sous pression et/ou à chaud.

8. Élément de semelle selon l'une des revendications 1 à 7, **caractérisée en ce que** l'élément de semelle intérieure comporte, sur une surface (4), une première indication dimensionnelle supplémentaire (9) destinée au positionnement d'une couverture,
ladite première indication dimensionnelle supplémentaire (9) indiquant une distance par rapport au bord de l'élément de semelle intérieure.

9. Élément de semelle selon l'une des revendications 1 à 8, **caractérisée en ce que** l'élément de semelle intérieure comporte, sur une surface (4), un adhésif ou un agent de liaison destiné à permettre la mise en place d'une couverture sur la surface (4) de l'élément de semelle intérieure.

10. Élément de semelle selon l'une des revendications 1 à 9, **caractérisée en ce que** l'élément de semelle intérieure comporte, sur une surface (4), un adhésif ou un agent de liaison destiné à permettre la mise en place d'un noyeau sur la surface (4) de l'élément de semelle intérieure.

11. Semelle intérieure comportant un élément de semelle intérieure et une couverture appliquée sur ledit élément de semelle intérieure, selon l'une des revendications 1 à 10.

12. Semelle intérieure selon la revendication 11, **caractérisée en ce que** la couverture comporte une deuxième ligne de centrage.
